# EUROPÄISCHE PATENTSCHRIFT

(11) **EP 2 491 968 B1**
(45) Veröffentlichungstag und Bekanntmachung des Hinweises auf die Patenterteilung: **17.09.2014**
(21) Anmeldenummer: 11155722.9
(22) Anmeldetag: 24.02.2011
(51) Int. Cl.: A61M 3/02, F04B 17/04

(54) **Pumpvorrichtung für sterile Fluide und Pumpensystem mit einer solchen Pumpvorrichtung**
Pump device for sterile fluids and pump system with such a pump device
Dispositif de pompe pour fluide stérile et système de pompe doté d'un tel dispositif de pompe

(43) Veröffentlichungstag der Anmeldung: 29.08.2012
(73) Patentinhaber: ERBE Elektromedizin GmbH, 72072 Tübingen (DE)
(72) Erfinder: Bartel, Volker, 72411, Bodelshausen (DE)
(74) Vertreter: Bohnenberger, Johannes

(56) Entgegenhaltungen:
- EP-A1- 1 764 504
- WO-A1-2008/110187
- DE-A1- 3 301 741
- DE-A1- 10 348 832
- US-A- 4 389 169
- US-A- 5 620 414

## Beschreibung

Die Erfindung betrifft eine Pumpvorrichtung für sterile Fluide zum Pumpen des sterilen Fluids von einem Reservoir oder dergleichen Quelle zu einem chirurgischen Instrument, insbesondere einem Instrument für die Wasserstrahlchirurgie oder dergleichen Verbraucher. Darüber hinaus betrifft die Erfindung ein Pumpensystem mit einer solchen Pumpvorrichtung.

In der Wasserstrahlchirurgie bestehen im Allgemeinen folgende Anforderungen:
- Die Sterilität des Fluids muss gewährleistet bleiben, da es nicht nur mit dem Körper eines Patienten in Kontakt gelangt, sondern auch teilweise im Körper verbleibt;
- der geförderte Fluss bzw. der dafür erforderliche Druck muss reproduzierbar einstellbar sein, um einen reproduzierbaren Gewebeeffekt zu erzielen;
- die Bedienungsperson muss frei sein in der Dauer der Einzelaktivierung einer Fluidabgabe;
- die Aktivierung des Instrumentes muss im Wesentlichen verzögerungsfrei begonnen und beendet werden können, d.h. der Druckaufbau muss schnell erfolgen und ein Nachtropfen darf nicht auftreten;
- Applikatoren müssen unproblematisch auswechselbar sein.

Chirurgische Wasserstrahlschneideinrichtungen und dazu gehörige Systeme zum Druckaufbau sind an sich bekannt. Für die Wasserstrahlchirurgie wird eine Pumpe benötigt, die einen Druck von 10 bis 80 bar erzeugt. Das Pumpensystem muss eine sterile Schnittstelle haben, damit das Fluid (z.B. Kochsalzlösung) unter sterilen Bedingungen eingesetzt werden kann.

Die DE 103 48 832 A1 beschreibt eine Pumpvorrichtung für sterile Fluide. Diese besitzt mindestens zwei Pumpen, die jeweils einen Kolben, einen Zylinder, eine Kolbenstange und verschiedene Pumpenleitungen aufweisen, die über Ausstoßventile und ein Klemmventil mit einem chirurgischen Instrument verbunden sind. Die Pumpenleitungen sind über Ansaugleitungen und Ansaugventile mit einer Ansaugleitung verbunden, die über eine lösbare Kupplung mit einer Quelle verbindbar ist. Die Kolbenstangen sind über eine erste Kupplung bzw. eine zweite Kupplung mit einem ersten Motor bzw. zweiten Motor lösbar verbunden. Eine Steuerung steuert die Motoren, so dass eine möglichst gleichmäßige Förderleistung erzielt wird. Dieses Pumpensystem hat jedoch eine aufwändige Mechanik, so dass es relativ kostenintensiv ist.

Eine Vorrichtung der hier angesprochenen Art ist auch aus der US 4,389,169 A bekannt.

Der Erfindung liegt die Aufgabe zu Grunde, eine kostengünstige Pumpvorrichtung für sterile Fluide und ein dazugehöriges Pumpensystem anzugeben.

Diese Aufgabe wird durch eine Pumpvorrichtung mit den Merkmalen von Anspruch 1 und eine Pumpvorrichtungsaufnahme mit den Merkmalen des Anspruchs 12 gelöst. Ausführungsformen sind in den abhängigen Ansprüchen angegeben.

Die Erfindung umfasst eine Pumpvorrichtung für sterile Fluide zum Pumpen des sterilen Fluids von einem Reservoir oder dergleichen Quelle zu einem chirurgischen Instrument, insbesondere einem Instrument für die Wasserstrahlchirurgie oder dergleichen Verbraucher. Die Pumpvorrichtung weist einen beweglichen Pumpenaktuator zum Ansaugen des Fluids in einem Ansaugzyklus und zum Ausstoßen des Fluids in einem Ausstoßzyklus auf. Zusätzlich werden eine erste Ventileinrichtung, die im Ansaugzyklus offen und im Ausstoßzyklus geschlossen ist, und eine zweite Ventileinrichtung, die im Ansaugzyklus geschlossen und im Ausstoßzyklus offen ist, bereitgestellt. Erfindungsgemäß umfasst der Pumpenaktuator ein magnetisierbares Material, insbesondere ein Metall, und ist durch eine elektromagnetische Spule berührungslos antreibbar, wobei die Pumpvorrichtung als Einwegprodukt ausgestaltet ist und einen Fixierring zum Positionieren der Pumpvorrichtung in einer Pumpenvorrichtungsaufnahme aufweist.

Durch diese spezifische Form des Antriebs kann die Mechanik der Pumpvorrichtung gegenüber dem Stand der Technik stark vereinfacht werden, so dass sich Kostenvorteile ergeben.

Die Pumpvorrichtung ist als Einwegprodukt ausgestaltet und umfasst auch entsprechende Anschlussschläuche und/oder das chirurgische Instrument. Durch die Ausgestaltung als Einwegprodukt wird ein hohes Maß an Sterilität erreicht. Nach einer Operation mit dem chirurgischen Instrument kann das Einwegprodukt einfach entsorgt werden.

Vorzugsweise ist der Pumpenaktuator zum Einstellen eines Nullpunktes zwischen einer ersten und zweiten Feder eingespannt. Auf diese Weise muss die elektromagnetische Spule nur dann bestromt werden, wenn der Pumpenaktuator aus diesem Nullpunkt abgelenkt werden soll.

Die erste und/oder zweite Ventileinrichtung können als Kugelventil mit einer Kugel und einer Ventilfeder ausgestaltet sein. Kugelventile sind eine einfache, verlässliche und kostengünstige Form von Ventilen.

Vorzugsweise ist die erste Ventileinrichtung in einer Druckkammer angeordnet. In einer Ausführungsform ragt der einen Kolben bildende Pumpenaktuator in diese Druckkammer hinein, wobei um den Pumpenaktuator ein Dichtring vorgesehen ist, um die Druckkammer flüssigkeitsdicht abzuschließen. Durch die Bewegung des Pumpenaktuators in der Druckkammer kann mit Hilfe der ersten Ventileinrichtung ein Druck aufgebaut werden, so dass das Fluid gepumpt wird.

In einer Ausführungsform weist der Pumpenaktuator ein rohrförmiges Element auf, bzw. ist als rohrförmiger Kolben ausgebildet, durch den während eines Ansaugvorgangs das Fluid fließt. Die Kugel der ersten Ventileinrichtung kann im Ausstoßzyklus die Öffnung des rohrförmigen Elements verschließen und im Ansaugzyklus die Öffnung des rohrförmigen Elements öffnen. Auf diese Weise wird besonders einfach und klein bauend ein entsprechender Pumpvorgang ermöglicht.

Die erste und zweite Ventileinrichtung sind vorzugsweise gemeinsam in einem Bauelement angeordnet. Auch der Pumpenaktuator kann in demselben Bauelement angeordnet sein. Dadurch ergibt sich eine besonders kompakte und kostengünstige Bauform.

Zusätzlich umfasst die vorliegende Erfindung ein Pumpensystem mit einer erfindungsgemäßen Pumpvorrichtung und einer Pumpvorrichtungsaufnahme, die mindestens eine elektromagnetische Spule umfasst. Das Pumpensystem umfasst eine Steuereinrichtung zum Steuern einer Frequenz eines Magnetisierungsstroms, mit dem die mindestens eine elektromagnetische Spule betrieben wird. Mit diesem Pumpensystem wird erreicht, dass die Pumpvorrichtung, die hohen Sterilitätsanforderungen genügen muss, als Einwegprodukt ausgestaltet werden kann, während die Bestandteile, die recht teuer sind und diesen hohen Sterilitätsanforderungen nicht genügen müssen, in der Pumpvorrichtungsaufnahme angeordnet sind, die wiederverwendet wird. Dadurch ergeben sich Kostenvorteile. Die Steuereinrichtung kann durch Steuern der Frequenz des Magnetisierungsstroms der mindestens einen elektromagnetischen Spule den Durchfluss durch die Pumpvorrichtung steuern.

In einer Ausführungsform weist die Pumpvorrichtung einen Fixierring und die Pumpvorrichtungsaufnahme eine Fixiernut auf. Beim Einlegen der Pumpvorrichtung in die Pumpvorrichtungsaufnahme greift der Fixierring in die Fixiernut ein. Auf diese Weise wird die Pumpvorrichtung in der Pumpvorrichtungsaufnahme fixiert, so dass eine verlässliche Verbindung zwischen der Pumpvorrichtung und der Pumpvorrichtungsaufnahme erreicht wird.

Nachfolgend werden bevorzugte Ausführungsformen der Erfindung anhand von Abbildungen näher erläutert. Hierbei zeigen
- Fig. 1: eine schematische Schnittdarstellung durch eine erste Ausführungsform einer erfindungsgemäßen Pumpvorrichtung;
- Fig. 2: die Pumpvorrichtung aus Fig. 1 im Ausstoßzyklus;
- Fig. 3: die Pumpvorrichtung aus Fig. 1 im Ansaugzyklus;
- Fig. 4: eine schematische Explosionsdarstellung einer Ausführungsform einer erfindungsgemäßen Pumpvorrichtung mit einer Ausführungsform einer erfindungsgemäßen Pumpvorrichtungsaufnahme;
- Fig. 5: die Pumpvorrichtung und Pumpvorrichtungsaufnahme aus Fig. 4 in zusammengesetzter Form; und
- Fig. 6: eine schematische Darstellung eines möglichen Einsatzumfeldes des Pumpensystems aus Fig. 5.

In der nachfolgenden Beschreibung werden für gleiche und gleich wirkende Teile dieselben Bezugsziffern verwendet.

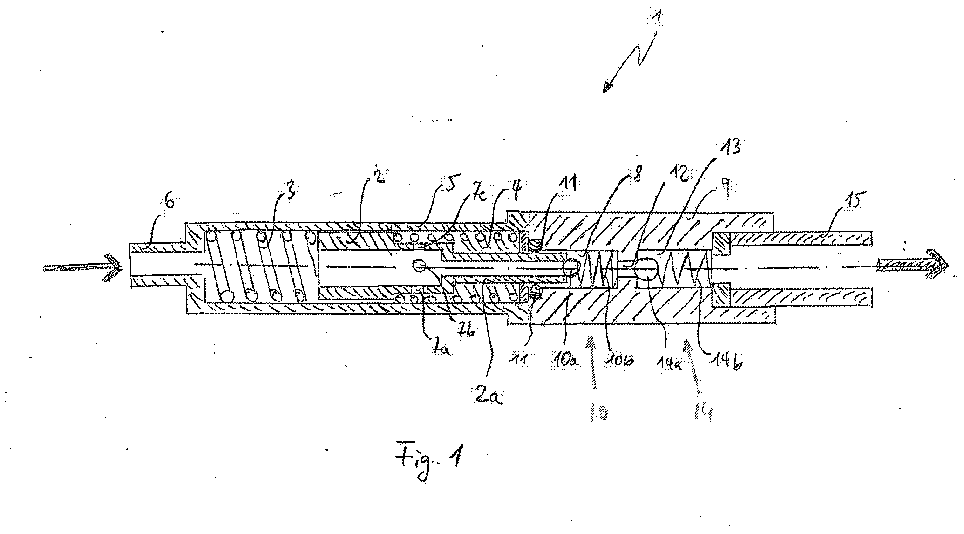

Eine erste Ausführungsform einer erfindungsgemäßen Pumpvorrichtung 1 ist in Fig. 1 in einer schematischen Schnittdarstellung gezeigt. Diese umfasst einen beweglichen Pumpenaktuator 2 zum Ansaugen eines Fluids in einem Ansaugzyklus und zum Ausstoßen des Fluids in einem Ausstoßzyklus, der zwischen einer ersten Feder 3 und einer zweiten Feder 4 eingespannt ist, um seinen Nullpunkt einzustellen. Der Pumpenaktuator 2 sowie die zwei Federn 3 und 4 befinden sich weitestgehend in einem ersten Abschnitt 5 der Pumpvorrichtung, die über einen Anschluss 6 mit einem Reservoir eines sterilen Fluids verbunden ist. In dem Pumpenaktuator 2 befinden sich verschiedene Löcher 7a, 7b, 7c, um innerhalb des ersten Abschnitts 5 einen Druckausgleich zwischen dem Fluid innerhalb des Pumpenaktuators und außerhalb des Pumpenaktuators zu ermöglichen.

Auf einer Seite ragt der Pumpenaktuator 2 in eine Druckkammer 8 hinein. Diese Druckkammer 8 ist in einem zweiten Abschnitt 9 der Pumpvorrichtung angeordnet. In der Druckkammer 8 befindet sich eine erste Ventileinrichtung 10, die im Ansaugzyklus offen und im Ausstoßzyklus geschlossen ist. Diese erste Ventileinrichtung 10 ist in diesem Ausführungseispiel als ein Kugelventil ausgestaltet und umfasst eine Kugel 10a sowie eine Ventilfeder 10b. Der Pumpenaktuator 2 weist ein rohrförmiges Element 2a auf, dessen Öffnung im Bereich der Druckkammer 8 im Ausstoßzyklus durch die Kugel 10a verschlossen wird. Auf der Seite zum ersten Abschnitt 5 hin ist um den Pumpenaktuator ein Dichtring 11 vorgesehen, der die Druckkammer 8 flüssigkeitsdicht abschließt.

Der durch die Federn 3 und 4 definierte Nullpunkt entspricht der äußersten Ausgangsposition, in welcher das rohrförmige Element 2a immer noch im Dichtring 11 steckt.

Durch eine Vorwärtsbewegung des Pumpenaktuators 2 (in Fig. 1 nach rechts) wird das Fluid aus der Druckkammer 8 über eine Passage 12 in eine Ausstoßkammer 13 gepumpt. In der Ausstoßkammer 13 befindet sich eine zweite Ventileinrichtung 14, die im Ansaugzyklus geschlossen und im Ausstoßzyklus offen ist. Die zweite Ventileinrichtung 14 ist hier wiederum als Kugelventil ausgestaltet und weist eine Kugel 14a und eine Ventilfeder 14b auf. Das Fluid verlässt die Ausstoßkammer 13 über einen Anschluss 15 in Richtung des chirurgischen Instruments.

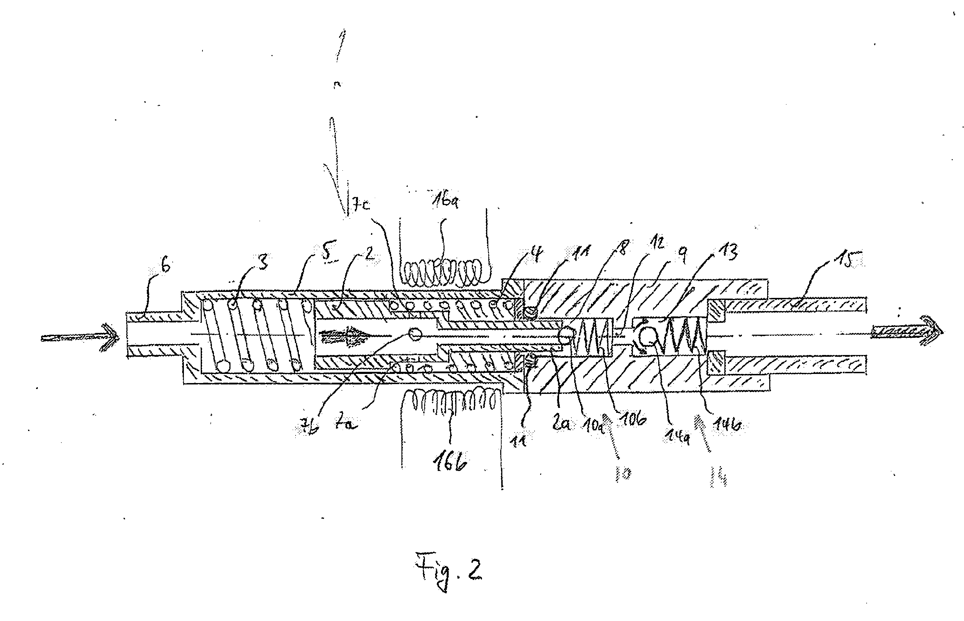

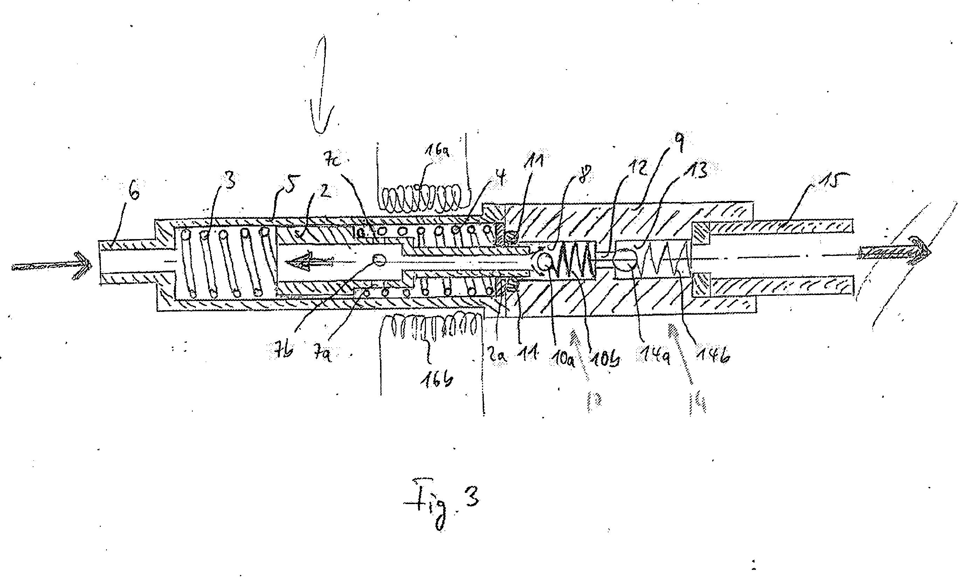

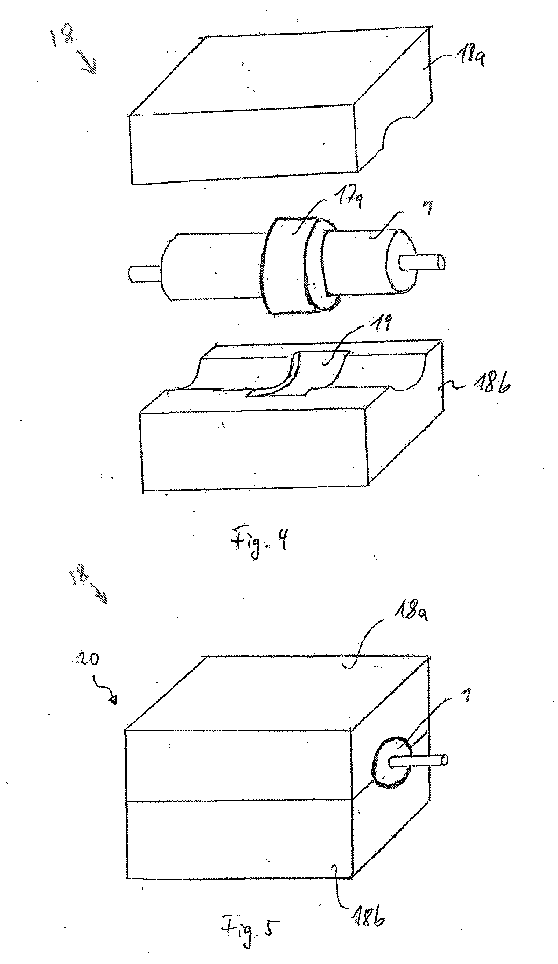

Wie später mit Bezug auf Fig. 4 erläutert wird, kann die Pumpvorrichtung 1 in eine Pumpvorrichtungsaufnahme eingelegt werden, die mindestens eine elektromagnetische Spule umfasst. Die Fig. 2 und 3 illustrieren eine Ausführungsform, bei der zwei Spulen 16a und 16b vorgesehen sind. Wenn die Pumpvorrichtung 1 in die Pumpvorrichtungsaufnahme eingelegt ist, befinden sich die beiden Spulen 16a und 16b in unmittelbarer Nähe des Pumpenaktuators 2, so dass diese, wenn sie bestromt werden, den Pumpenaktuator 2 in Richtung des zweiten Abschnittes 9 bewegen können. Sind die Spulen stromlos, treibt die zweite Feder 4 den Pumpenaktuator 2 zurück zu seinem Nullpunkt. Somit kann durch Ein- und Ausschalten eines Stromes, der durch die beiden Spulen 16a und 16b fließt und mittels der Kraft der Feder 4, der Pumpenaktuator 2 abwechselnd in Richtung des zweiten Abschnittes 9 und anschließend zurück in Richtung des ersten Abschnittes 5 bewegt werden.

Fig. 2 illustriert eine Situation, in der Strom durch die beiden Spulen 16a und 16b fließt, so dass der Pumpenaktuator 2 in Richtung des zweiten Abschnittes 9 bewegt wird. Wie der Fig. 2 zu entnehmen ist, verschließt dabei die Kugel 10a die Öffnung des rohrförmigen Elements 2a, die im Bereich der Druckkammer 8 angeordnet ist, so dass durch die Bewegung des Pumpenaktuators 2 Fluid aus der Druckkammer 8 über die Passage 12 in die Ausstoßkammer 13 gefördert wird. Bei diesem Fördervorgang, hebt sich die Kugel 14a der zweiten Ventileinrichtung 14 von der Öffnung der Passage 12 ab.

Fig. 3 zeigt die Situation im Ansaugzyklus, bei dem die Spulen 16a und 16b stromlos sind. Die Kraft der zweiten Feder 4 bewegt dabei den Pumpenaktuator 2 in Richtung des ersten Abschnittes 5. Dadurch hebt sich die Kugel 10a der ersten Ventileinrichtung 10 von der Öffnung des rohrförmigen Elements 2a des Pumpenaktuators 2 ab, so dass Fluid in die Druckkammer 8 gelangen kann. Gleichzeitig drückt die Ventilfeder 14b der zweiten Ventileinrichtung 14 die Kugel 14a gegen die Öffnung der Passage 12, so dass verhindert wird, dass Fluid aus der Ausstoßkammer 13 zurück in die Druckkammer 8 fließt. Durch wiederholtes Durchlaufen des in Fig. 2 dargestellten Ausstoßzyklus und des in Fig. 3 illustrierten Ansaugzyklus kann mittels der Pumpvorrichtung und der beiden Spulen 16a und 16b ein Pumpvorgang durchgeführt werden.

Fig. 4 illustriert, wie eine erfindungsgemäße Pumpvorrichtung 1 in eine Pumpvorrichtungsaufnahme 18 eingelegt werden kann. Die Pumpvorrichtungsaufnahme 18 besteht dabei aus einer oberen Hälfte 18a und einer unteren Hälfte 18b. Die Pumpvorrichtung 1 umfasst einen Fixierring 17a. Dieser greift beim Einlegen in eine Fixiernut 19 ein, die sich sowohl in der oberen Hälfte 18a als auch der unteren Hälfte 18b befindet. Die Pumpvorrichtung 1 wird derart in die untere Hälfte 18b der Pumpvorrichtungsaufnahme 18 gelegt, dass der Fixierring 17a in der Fixiernut 19 positioniert wird. Anschließend wird die obere Hälfte 18a auf die untere Hälfte 18b der Pumpvorrichtungsaufnahme 18 aufgesetzt, um die Pumpvorrichtungsaufnahme 18 zu verschließen. Wie bereits mit Bezug auf die Fig. 2 und 3 erläutert wurde, kann sowohl die obere Hälfte 18a als auch die untere Hälfte 18b der Pumpvorrichtungsaufnahme 18 eine elektromagnetische Spule 16a und 16b umfassen.

Die Fig. 5 zeigt die obere Hälfte 18a und die untere Hälfte 18b der Pumpvorrichtungsaufnahme 18 aus Fig. 4 in einem zusammengesetzten Zustand, so dass sich die Pumpvorrichtung 1 zwischen den beiden Hälften 18a, 18b der Pumpvorrichtungsaufnahme 18 befindet und ein Pumpensystem 20 gebildet wird.

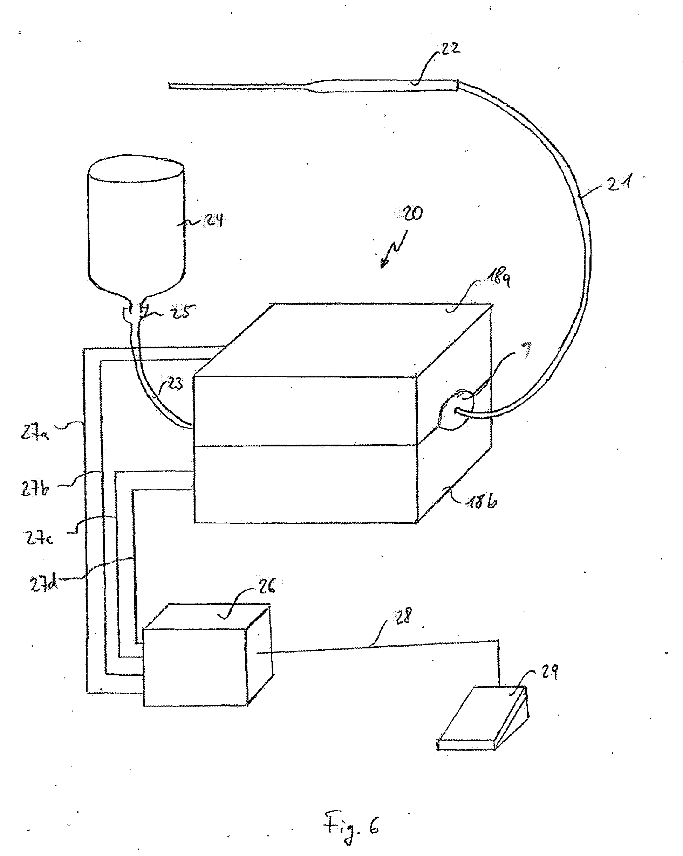

Das Pumpensystem 20 aus Fig. 5 wird in Fig. 6 in einem Anwendungsszenario dargestellt. An der Ausgangsseite der Pumpvorrichtung 1 befindet sich ein Schlauch 21, an dessen Ende ein chirurgisches Instrument 22 für die Wasserstrahlchirurgie angeordnet ist. Über einen Eingangsschlauch 23 wird die Pumpvorrichtung 1 mit einem Fluid (z.B. Kochsalzlösung) versorgt, das einem Reservoir 24 über eine lösbare Kupplung 25 entnommen wird. Eine Steuereinrichtung 26 kann dabei über elektrische Leitungen 27a, 27b, 27c, 27d die in der Pumpvorrichtungsaufnahme 18 befindlichen Spulen derart mit Strom versorgen, dass das Pumpverhalten, insbesondere der Druck (entsprechend dem Magnetisierungsstrom) und die Flussrate (entsprechend der Bestromungsfrequenz), der Pumpvorrichtung 1 gesteuert wird. Die Steuereinrichtung 26 kann dabei Eingangssignale über eine elektrische Leitung 28 empfangen, die beispielsweise durch einen Fußschalter 29 erzeugt werden, der von einem behandelnden Arzt betätigt wird.

Die beschriebene Ausführungsform des Pumpensystems weist eine einfache Mechanik auf und ist einfach steuerbar. Dadurch ergeben sich gegenüber dem Stand der Technik erhebliche Kostenvorteile. Die Pumpvorrichtung kann kostengünstig als steriles Einwegprodukt hergestellt werden.

Die mit Bezug auf die Abbildungen ausgeführten Erläuterungen sind rein illustrativ und nicht beschränkend zu verstehen. An den gezeigten Ausführungsformen können selbstverständlich Änderungen vorgenommen werden, ohne den Schutzbereich der Erfindung zu verlassen, wie er in den beigefügten Ansprüchen festgelegt ist.

Die vorliegende Erfindung betrifft eine Pumpvorrichtung (1) für sterile Fluide und ein Pumpensystem (20) mit einer solchen Pumpvorrichtung. Die Pumpvorrichtung (1) umfasst einen beweglichen Pumpenaktuator (2) zum Ansaugen eines Fluids in einem Ansaugzyklus und zum Ausstoßen des Fluids in einem Ausstoßzyklus. Eine erste Ventileinrichtung (10) ist im Ansaugzyklus offen und im Ausstoßzyklus geschlossen, während eine zweite Ventileinrichtung (14) im Ansaugzyklus geschlossen und im Ausstoßzyklus offen ist. Der Pumpenaktuator (2) umfasst ein magnetisierbares Material, insbesondere ein Metall, und ist durch eine elektromagnetische Spule (16a, 16b) berührungslos antreibbar.

### Bezugszeichenliste

- 1: Pumpvorrichtung
- 2: Pumpenaktuator
- 2a: rohrförmiges Element
- 3: erste Feder
- 4: zweite Feder
- 5: erster Abschnitt
- 6: Anschluss
- 7a-7c: Loch
- 8: Druckkammer
- 9: zweiter Abschnitt
- 10: erste Ventileinrichtung
- 10a: Kugel von 10
- 10b: Feder von 10
- 11: Dichtring
- 12: Passage
- 13: Ausstoßkammer
- 14: zweite Ventileinrichtung
- 14a: Kugel der zweiten Ventileinrichtung
- 14b: Ventilfeder der zweiten Ventileinrichtung
- 15: Anschluss
- 16a: elektromagnetische Spule
- 16b: elektromagnetische Spule
- 17a: Fixierring
- 18: Pumpvorrichtungsaufnahme
- 18a: obere Hälfte von 18
- 18b: untere Hälfte von 18
- 19: Fixiernut
- 20: Pumpensystem
- 21: Schlauch
- 22: chirurgisches Instrument
- 23: Schlauch
- 24: Fluidreservoir
- 25: lösbare Kupplung
- 26: Steuereinrichtung
- 27a-27d: elektrische Leitung
- 28: elektrische Leitung
- 29: Fußschalter

## Patentansprüche

1. Pumpvorrichtung (1) für sterile Fluide zum Pumpen des sterilen Fluids von einem Reservoir (24) oder dergleichen Quelle zu einem chirurgischen Instrument (22), insbesondere einem Instrument für die Wasserstrahlchirurgie oder dergleichen Verbraucher, umfassend
einen beweglichen Pumpenaktuator (2) zum Ansaugen des Fluids in einem Ansaugzyklus und zum Ausstoßen des Fluids in einem Ausstoßzyklus,
eine erste Ventileinrichtung (10), die im Ansaugzyklus offen und im Ausstoßzyklus geschlossen ist, und
eine zweite Ventileinrichtung (14), die im Ansaugzyklus geschlossen und im Ausstoßzyklus offen ist, wobei
der Pumpenaktuator (2) ein magnetisierbares Material, insbesondere ein Metall, umfasst und durch eine elekromagnetische Spule (16a, 16b) berührungslos antreibbar ist,
**dadurch gekennzeichnet, dass**
die Pumpvorrichtung (1) als Einwegprodukt ausgestaltet ist und einen Fixierring (17a) zum Positionieren der Pumpvorrichtung (1) in einer Pumpenvorrichtungsaufnahme (18) aufweist.

2. Pumpvorrichtung nach Anspruch 1,
**dadurch gekennzeichnet, dass**
der Pumpenaktuator (2) zum Einstellen eines Nullpunktes zwischen einer ersten Feder (3) und zweiten Feder (4) eingespannt ist.

3. Pumpvorrichtung nach einem der vorhergehenden Ansprüche,
**dadurch gekennzeichnet, dass**
die erste Ventileinrichtung (10) und/oder zweite Ventileinrichtung (14) als Kugelventil mit einer Kugel (10a, 14a) und einer Ventilfeder (10b, 14b) ausgestaltet sind.

4. Pumpvorrichtung nach einem der vorhergehenden Ansprüche, insbesondere nach Anspruch 3,
**dadurch gekennzeichnet, dass**
die erste Ventileinrichtung (10) in einer Druckkammer (8) angeordnet ist.

5. Pumpvorrichtung nach Anspruch 4,
**dadurch gekennzeichnet, dass**
der Pumpenaktuator (2) in die Druckkammer (8) hineinragt, wobei um den Pumpenaktuator (2) ein Dichtring (11) vorgesehen ist, um die Druckkammer (8) flüssigkeitsdicht abzuschließen.

6. Pumpvorrichtung nach einem der vorhergehenden Ansprüche,
**dadurch gekennzeichnet, dass**
der Pumpenaktuator (2) ein rohrförmiges Element (2a) aufweist, durch das während des Ansaugzyklus das Fluid fließt.

7. Pumpvorrichtung nach einem der Ansprüche 3-6,
**dadurch gekennzeichnet, dass**
die Kugel (10a) der ersten Ventileinrichtung (10) im Ausstoßzyklus die Öffnung des rohrförmigen Elements (2a) verschließt und im Ansaugzyklus die Öffnung des rohrförmigen Elements (2a) öffnet.

8. Pumpvorrichtung nach einem der vorhergehenden Ansprüche,
**dadurch gekennzeichnet, dass**
die erste und zweite Ventileinrichtung (10, 14) gemeinsam in einem Bauelement angeordnet sind.

9. Pumpvorrichtung nach Anspruch 8,
**dadurch gekennzeichnet, dass**
der Pumpenaktuator (2) in dem Bauelement angeordnet ist.

10. Pumpvorrichtung (1) nach einem der vorhergehenden Ansprüche,
**dadurch gekennzeichnet, dass**
die Pumpvorrichtung Anschlussschläuche (21, 23) umfasst.

11. Pumpvorrichtung (1) nach einem der vorhergehenden Ansprüche,
**dadurch gekennzeichnet, dass**
die Pumpvorrichtung das chirurgische Instrument (22) umfasst.

12. Pumpvorrichtungsaufnahme (18) zur Aufnahme einer Pumpvorrichtung (1) für sterile Fluide zum Pumpen des sterilen Fluids von einem Reservoir (24) oder dergleichen Quelle zu einem chirurgischen Instrument (22), insbesondere einem Instrument für die Wasserstrahlchirurgie oder dergleichen Verbraucher, umfassend
eine obere Hälfte (18a) und eine untere Hälfte (18b), wobei die untere Hälfte (18b) und die obere Hälfte (18a) eine Fixiernut (19) zur Aufnahme eines Fixierrings (17a) der Pumpvorrichtung (1) aufweisen, sodass der Fixierring (17a) beim Einlegen der Pumpvorrichtung (1) in die Pumpvorrichtungsaufnahme (18) in die Fixiernut (19) eingreift, um so die Pumpvorrichtung (1) in der Pumpvorrichtungsaufnahme (18) zu fixieren, wobei die Pumpvorrichtungsaufnahme (18) mindestens eine elektromagnetische Spule (16a, 16b) umfasst.

13. Pumpensystem (20) mit einer Pumpvorrichtung (1) nach einem der vorhergehenden Ansprüche 1 bis 11 und einer Pumpvorrichtungsaufnahme (18) nach Anspruch 12.

14. Pumpensystem (20) nach Anspruch 13,
**gekennzeichnet durch**
eine Steuereinrichtung (26) zum Steuern einer Frequenz eines Magnetisierungsstroms, mit dem die mindestens eine elektromagnetische Spule (16a, 16b) betrieben wird.

## Claims

1. Pump device (1) for sterile fluids for pumping the sterile fluid from a reservoir (24) or similar source to a surgical instrument (22), in particular an instrument for water jet surgery or similar consumers, comprising
a movable pump actuator (2) for suction of the fluid in an intake cycle and for ejection of the fluid in a discharge cycle,
a first valve (10), which is open in the intake cycle and closed in the discharge cycle, and
a second valve (14), which is closed in the intake cycle and open in the discharge cycle, wherein
the pump actuator (2) comprises a magnetizable material, in particular a metal, and can be driven contactlessly by an electromagnetic coil (16a, 16b),
**characterized in that** the pump device (1) is designed as a disposable product and has a fixing ring (17a) for positioning the pump device (1) in a pump device holder (18).

2. Pump device according to Claim 1, **characterized in that** the pump actuator (2) is clamped between a first spring (3) and second spring (4) in order to set a zero point.

3. Pump device according to one of the preceding claims, **characterized in that** the first valve (10) and/or second valve (14) are each designed as a ball valve with a ball (10a, 14a) and a valve spring (10b, 14b).

4. Pump device according to one of the preceding claims, in particular according to Claim 3, **characterized in that** the first valve (10) is arranged in a pressure chamber (8).

5. Pump device according to Claim 4, **characterized in that** the pump actuator (2) protrudes into the pressure chamber (8), wherein a sealing ring (11) is provided around the pump actuator (2) in order to close off the pressure chamber (8) in a leaktight manner.

6. Pump device according to one of the preceding claims, **characterized in that** the pump actuator (2) has a tubular element (2a) through which the fluid flows during the intake cycle.

7. Pump device according to one of Claims 3-6, **characterized in that** the ball (10a) of the first valve (10) closes the opening of the tubular element (2a) in the discharge cycle and opens the opening of the tubular element (2a) in the intake cycle.

8. Pump device according to one of the preceding claims, **characterized in that** the first and second valves (10, 14) are arranged together in a structural element.

9. Pump device according to Claim 8, **characterized in that** the pump actuator (2) is arranged in the structural element.

10. Pump device (1) according to one of the preceding claims, **characterized in that** the pump device comprises connector hoses (21, 23).

11. Pump device (1) according to one of the preceding claims, **characterized in that** the pump device comprises the surgical instrument (22).

12. Pump device holder (18) for holding a pump device (1) for sterile fluids for pumping the sterile fluid from a reservoir (24) or similar source to a surgical instrument (22), in particular an instrument for water jet surgery or similar consumers, comprising
an upper half (18a) and a lower half (18b), wherein the lower half (18b) and the upper half (18a) have a fixing groove (19) for receiving a fixing ring (17a) of the pump device (1), such that, when the pump device (1) is placed in the pump device holder (18), the fixing ring (17a) engages in the fixing groove (19) in order thereby to fix the pump device (1) in the pump device holder (18), wherein the pump device holder (18) comprises at least one electromagnetic coil (16a, 16b).

13. Pump system (20) with a pump device (1) according to one of Claims 1 to 11 and with a pump device holder (18) according to Claim 12.

14. Pump system (20) according to Claim 13, **characterized by** a controller (26) for controlling a frequency of a magnetizing current with which the at least one electromagnetic coil (16a, 16b) is operated.

## Revendications

1. Dispositif de pompe (1) pour fluides stériles pour pomper le fluide stérile d'un réservoir (24) ou d'une source similaire dans un instrument chirurgical (22), en particulier un instrument pour la chirurgie par jet d'eau ou un consommateur similaire, comprenant
un actionneur de pompe mobile (2) pour aspirer le fluide dans un cycle d'aspiration et pour éjecter le fluide dans un cycle d'éjection,
un premier dispositif de soupape (10) qui, dans le cycle d'aspiration, est ouvert et qui, dans le cycle d'éjection, est fermé, et
un deuxième dispositif de soupape (14) qui, dans le cycle d'aspiration, est fermé et qui, dans le cycle d'éjection, est ouvert,
l'actionneur de pompe (2) comprenant un matériau magnétisable, en particulier un métal et pouvant être entraîné sans contact par une bobine électromagnétique (16a, 16b),
**caractérisé en ce que**
le dispositif de pompe (1) est configuré sous forme de produit à usage unique et présente une bague de fixation (17a) pour positionner le dispositif de pompe (1) dans un logement de dispositif de pompe (18).

2. Dispositif de pompe selon la revendication 1,
**caractérisé en ce que**
l'actionneur de pompe (2), pour ajuster un point zéro, est inséré entre un premier ressort (3) et un deuxième ressort (4).

3. Dispositif de pompe selon l'une quelconque des revendications précédentes,
**caractérisé en ce que**
le premier dispositif de soupape (10) et/ou le deuxième dispositif de soupape (14) sont configurés sous forme de soupape sphérique avec une bille (10a, 14a) et un ressort de soupape (10b, 14b).

4. Dispositif de pompe selon l'une quelconque des revendications précédentes, en particulier selon la revendication 3,
**caractérisé en ce que**
le premier dispositif de soupape (10) est disposé dans une chambre de pression (8).

5. Dispositif de pompe selon la revendication 4,
**caractérisé en ce que**
l'actionneur de pompe (2) pénètre dans la chambre de pression (8), une bague d'étanchéité (11) étant prévue autour de l'actionneur de pompe (2), afin de fermer de manière étanche aux liquides la chambre de pression (8).

6. Dispositif de pompe selon l'une quelconque des revendications précédentes,
**caractérisé en ce que**
l'actionneur de pompe (2) présente un élément de forme tubulaire (2a) à travers lequel s'écoule le fluide pendant le cycle d'aspiration.

7. Dispositif de pompe selon l'une quelconque des revendications 3-6,
**caractérisé en ce que**
la bille (10a) du premier dispositif de soupape (10), dans le cycle d'éjection, ferme l'ouverture de l'élément de forme tubulaire (2a) et, dans le cycle d'aspiration, ouvre l'ouverture de l'élément de forme tubulaire (2a).

8. Dispositif de pompe selon l'une quelconque des revendications précédentes,
**caractérisé en ce que**
le premier et le deuxième dispositif de soupape (10, 14) sont disposés conjointement dans un élément structurel.

9. Dispositif de pompe selon la revendication 8, **caractérisé en ce que**
l'actionneur de pompe (2) est disposé dans l'élément structurel.

10. Dispositif de pompe (1) selon l'une quelconque des revendications précédentes,
**caractérisé en ce que**
le dispositif de pompe comprend des tuyaux de raccordement (21, 23).

11. Dispositif de pompe (1) selon l'une quelconque des revendications précédentes,
**caractérisé en ce que**
le dispositif de pompe comprend l'instrument chirurgical (22).

12. Logement de dispositif de pompe (18) pour recevoir un dispositif de pompe (1) pour fluides stériles pour pomper le fluide stérile d'un réservoir (24) ou d'une source similaire dans un instrument chirurgical (22), en particulier un instrument pour la chirurgie par jet d'eau ou un consommateur similaire, comprenant
une moitié supérieure (18a) et une moitié inférieure (18b), la moitié inférieure (18b) et la moitié supérieure (18a) présentant une rainure de fixation (19) pour recevoir une bague de fixation (17a) du dispositif de pompe (1), de telle sorte que la bague de fixation (17a), lors de l'insertion du dispositif de pompe (1) dans le logement de dispositif de pompe (18), vienne en prise dans la rainure de fixation (19), afin de fixer le dispositif de pompe (1) dans le logement de dispositif de pompe (18), le logement de dispositif de pompe (18) comprenant au moins une bobine électromagnétique (16a, 16b).

13. Système de pompe (20) comprenant un dispositif de pompe (1) selon l'une quelconque des revendications précédentes 1 à 11 et un logement de dispositif de pompe (18) selon la revendication 12.

14. Système de pompe (20) selon la revendication 13, **caractérisé par**
un dispositif de commande (26) pour commander une fréquence d'un courant de magnétisation avec lequel l'au moins une bobine électromagnétique (16a, 16b) est alimentée.
